(19) Europäisches Patentamt
European Patent Office
Office européen des brevets

(11) **EP 3 562 275 B1**

(12) **EUROPÄISCHE PATENTSCHRIFT**

(45) Veröffentlichungstag und Bekanntmachung des Hinweises auf die Patenterteilung:
**08.04.2020 Patentblatt 2020/15**

(51) Int Cl.:
*H05G 1/12* *(2006.01)* *H01F 27/28* *(2006.01)*
*H01F 27/32* *(2006.01)* *H01F 30/04* *(2006.01)*
*H01F 30/00* *(2006.01)*

(21) Anmeldenummer: **18168791.4**

(22) Anmeldetag: **23.04.2018**

(54) **HOCHSPANNUNGSTRANSFORMATOR**

HIGH VOLTAGE TRANSFORMER

TRANSFORMATEUR HAUTE TENSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.10.2019 Patentblatt 2019/44**

(73) Patentinhaber: **Siemens Healthcare GmbH 91052 Erlangen (DE)**

(72) Erfinder: **Waffler, Stefan 91054 Buckenhof (DE)**

(56) Entgegenhaltungen:
**DE-A1- 3 929 888 US-A- 4 481 654**

EP 3 562 275 B1

**Beschreibung**

Die Erfindung betrifft eine Schaltungsanordnung, eine Röntgeneinrichtung und einen Computertomographen.

Zu einer Versorgung einer Röntgenröhre mit Hochspannung werden typischerweise Hochspannungstransformatoren eingesetzt. Auf einer Kathodenseite liefert der Hochspannungstransformator beispielsweise eine relativ zu einem Erdpotential negative Hochspannung und auf einer Anodenseite eine relativ zu dem Erdpotential positive Hochspannung. Insbesondere eine daraus resultierende Hochspannung zwischen einer Anode der Röntgenröhre und einer Kathode der Röntgenröhre beschleunigt aus der Kathode emittierte Elektronen, welche üblicherweise auf der Anode auftreffen und dabei abgebremst werden, wodurch insbesondere Röntgenstrahlung für eine Bildgebung entsteht. Allerdings wird typischerweise ein gewisser Anteil der emittierten Elektronen an der Anode zurückgestreut.

Die zurückgestreuten Elektronen werden beispielsweise von einem geerdeten Metallmittelteil der Röntgenröhre eingefangen, wodurch sich insbesondere ein Differenzstrom in der Röntgenröhre einstellt. Daher wirkt die Röntgenröhre insbesondere als asymmetrische Last. Aufgrund des Differenzstroms der Röntgenröhre wird typischerweise der Hochspannungstransformator auf der Anodenseite und auf der Kathodenseite in Bezug auf einen jeweiligen Laststrom asymmetrisch belastet. In anderen Worten stellt sich zwischen der Anodenseite und der Kathodenseite ein Differenzstrom des Hochspannungstransformators ein, welcher zum Differenzstrom der Röntgenröhre proportional ist. Typischerweise korreliert also der Differenzstrom der Röntgenröhre mit dem Differenzstrom des Hochspannungstransformators. Der Differenzstrom des Hochspannungstransformators entsteht insbesondere dadurch, dass sich der Laststrom auf der Anodenseite und der Laststrom auf der Kathodenseite unterscheiden, insbesondere asymmetrisch sind. In diesem Fall folgt typischerweise eine Spannungsasymmetrie am Hochspannungstransformator. Allerdings ist ein herkömmlicher Hochspannungstransformator üblicherweise symmetrisch aufgebaut, weshalb sich am herkömmlichen Hochspannungstransformator aufgrund des Differenzstroms der Röntgenröhre die Spannungsasymmetrie einstellen kann. Die Spannungsasymmetrie folgt insbesondere daraus, dass sich insbesondere unterschiedliche Hochspannungen auf der Anodenseite und der Kathodenseite einstellen. Durch die Spannungsasymmetrie kann der Hochspannungstransformator und/oder die Röntgenröhre Schaden nehmen. Des Weiteren kann sich eine Qualität der Bildgebung verringern, falls der Hochspannungstransformator für die Röntgenröhre bei der Bildgebung verwendet wird.

Üblicherweise werden daher zwei voneinander unabhängige Hochspannungstransformatoren eingesetzt, wobei durch eine unabhängige Regelung der kathodenseitigen und anodenseitigen Hochspannungen die Spannungsasymmetrie verhindert werden kann. Dadurch steigen Herstellungs- und Wartungskosten erheblich an. Denn typischerweise müssen aufgrund der beiden Hochspannungstransformatoren noch weitere Einheiten, insbesondere Hochspannungswechselrichter und/oder zugehörige Schwingkreisdrosseln, ebenfalls mehrfach verbaut werden.

Eine alternative Möglichkeit ist ein Einsatz eines Mittelspannungstransformators mit nachgeschalteter Hochspannungskaskade, wodurch eine Komplexität einer Schaltungsanordnung mit dem Mittelspannungstransformator, der Hochspannungskaskade sowie der Röntgenröhre und damit ebenfalls die Kosten ansteigen.

Die US 4,481,654 A offenbart einen Hochspannungstransformator bestehend aus zwei Transformatoren mit übereinstimmender Resonanzfrequenz.

Der Erfindung liegt die Aufgabe zu Grunde, eine Schaltungsanordnung, eine Röntgeneinrichtung und einen Computertomographen anzugeben, bei welchen eine Spannungsasymmetrie aufgrund einer asymmetrischen Last verringert wird.

Die Aufgabe wird durch die Merkmale der unabhängigen Ansprüche gelöst. Vorteilhafte Ausgestaltungen sind in den Unteransprüchen beschrieben.

Bauartbedingt können sich bei elektronischen Schaltungen oder Bauelementen in einer realen Umgebung Abweichungen von den Vorgaben in einer idealen Umgebung ergeben. Wenn im Folgenden von Übereinstimmung, Entsprechung, Baugleichheit etc. gesprochen wird, beziehen sich diese Formulierungen in erster Linie auf eine Definition oder Dimensionierung der Schaltung oder des Bauelements in der idealen Umgebung. In der realen Umgebung können sich dennoch Abweichungen ergeben, obwohl diese Abweichung der Schaltung oder des Bauelements typischerweise nicht vorgesehen ist. Die ideale Umgebung kann beispielsweise eine Software zum Entwurf eines Hochspannungstransformators sein.

Die erfindungsgemäße Schaltungsanordnung weist

- eine Röntgenröhre, welche eine Kathode und eine Anode aufweist, und
- einen Hochspannungstransformator auf, welcher
- zumindest eine Primärwicklung,
- zumindest eine erste Sekundärwicklung, welche einen ersten Schwingkreis aufweist, wobei der erste Schwingkreis eine erste Streuinduktivität und eine erste Wicklungskapazität aufweist, welche gemeinsam eine erste Resonanzfrequenz einstellen, und
- zumindest eine zweite Sekundärwicklung aufweist, welche einen zweiten Schwingkreis aufweist, wobei der zweite Schwingkreis eine zweite Streuinduktivität und eine zweite Wicklungskapazität aufweist, welche gemeinsam eine zweite Resonanzfrequenz einstellen,
- wobei die erste Resonanzfrequenz und die zweite Resonanzfrequenz übereinstimmen,
- wobei sich die erste Wicklungskapazität und die zweite Wicklungskapazität betragsmäßig unterscheiden und
- wobei sich die erste Streuinduktivität und die zweite Streuinduktivität betragsmäßig unterscheiden,

dadurch gekennzeichnet,

- dass die zumindest eine erste Sekundärwicklung mit der Kathode elektrisch verbunden ist und
- dass die zumindest eine zweite Sekundärwicklung mit der Anode elektrisch verbunden ist.

Der Hochspannungstransformator bietet insbesondere folgende Vorteile: eine asymmetrische Last, insbesondere die Röntgenröhre, kann mit einer mittels des Hochspannungstransformator erzeugter Hochspannung betrieben werden. Vorteilhafterweise kann mittels des Hochspannungstransformators allein eine Reduzierung einer Spannungsasymmetrie erfolgen, wodurch ein Wartungsaufwand des Hochspannungstransformators vorzugsweise geringer ausfällt. Durch die Reduzierung der Spannungsasymmetrie können vorzugsweise bei einer Bildgebung Messdaten mit einer hohen Bildqualität erfasst werden, falls der Hochspannungstransformator bei der Bildgebung eingesetzt wird. Die Reduzierung der Spannungsasymmetrie erfolgt vorzugsweise ohne zusätzliche Bauelemente, welche typischerweise die asymmetrische Last ausgleichen sollen.

Der Hochspannungstransformator ist insbesondere vorteilhaft, weil der Hochspannungstransformator typischerweise eine höhere Leistungsdichte im Vergleich zu zwei herkömmlichen symmetrischen Hochspannungstransformatoren in Parallelschaltung aufweist.

Vorteilhafterweise kann durch das Abstimmen der ersten Resonanzfrequenz und der zweiten Resonanzfrequenz die Spannungsasymmetrie über einen Betriebsfrequenzbereich, insbesondere eines für die Hochspannungserzeugung eingesetzten Resonanzwandlers, gering gehalten werden.

Eine Ausführungsform sieht vor, dass sich in der zumindest einen ersten Sekundärwicklung ein erster Laststrom einstellt, wobei sich in der zumindest einen zweiten Sekundärwicklung ein zweiter Laststrom einstellt, wobei sich der erste Laststrom von dem zweiten Laststrom um einen Differenzstrom des Hochspannungstransformators unterscheidet, welcher zu einem Differenzstrom der Röntgenröhre proportional ist. Vorzugsweise ist der Differenzstrom des Hochspannungstransformators, insbesondere der erste Laststrom und der zweite Laststrom derart dimensioniert, dass die Spannungsasymmetrie am Hochspannungstransformator verringert wird. Besonders vorteilhafterweise entsprechen sich die kathodenseitige Hochspannung und die anodenseitige Hochspannung betragsmäßig, vorzugsweise trotz der asymmetrischen Last, insbesondere trotz des Differenzstroms der Röntgenröhre. Der Hochspannungstransformator gleicht also die Spannungsasymmetrie vorzugweise aus. Vorteilhafterweise ist der Hochspannungstransformator in Abhängigkeit von der asymmetrischen Last, insbesondere von der Röntgenröhre, ausgebildet. Der Hochspannungstransformator bietet also den Vorteil einer individuellen Anpassung an die asymmetrische Last.

Eine Ausführungsform sieht vor, dass die zumindest eine Primärwicklung einen ersten Zylinder mit einer ersten Längsachse bildet, wobei die zumindest eine erste Sekundärwicklung und die zumindest eine zweite Sekundärwicklung gemeinsam einen zweiten Zylinder mit einer zweiten Längsachse bilden, wobei die erste Längsachse parallel zur zweiten Längsachse ist und wobei der erste Zylinder relativ zum zweiten Zylinder entlang der ersten Längsachse versetzt angeordnet ist. Die versetzte Anordnung des ersten Zylinders relativ zum zweiten Zylinder ist insbesondere vorteilhaft, weil eine entsprechende bauliche Maßnahme einfach durchgeführt werden kann. Vorteilhafterweise sind mittels dieser Ausführungsform die Streuinduktivitäten präzise einstellbar.

Eine Ausführungsform sieht vor, dass sich ein Wicklungsdraht der zumindest einen ersten Sekundärwicklung von einem Wicklungsdraht der zumindest einen zweiten Sekundärwicklung gemäß zumindest einem Parameter der folgenden Liste unterscheidet:

- einer Dicke des Wicklungsdrahts,
- einer Dicke einer Lackisolationsschicht des Wicklungsdrahts,
- einer Dielektrizitätszahl der Lackisolationsschicht des Wicklungsdrahts. Eine Variation einer Ausführung des Wicklungsdrahts der zumindest einen ersten Sekundärwicklung von einer Ausführung des Wicklungsdrahts der zumindest einen zweiten Sekundärwicklung ist vorteilhaft, weil diese Variation mehrmals durchgeführt werden kann. Vorzugsweise kann die zumindest eine erste Sekundärwicklung und/oder die zumindest eine zweite Sekundärwicklung einfach ausgetauscht werden. Vorteilhafterweise sind mittels dieser Ausführungsform die Wicklungskapazitäten präzise einstellbar.

Eine Ausführungsform sieht vor, dass ein erster Durchmesser der zumindest einen ersten Sekundärwicklung kleiner ist als ein zweiter Durchmesser der zumindest einen zweiten Sekundärwicklung, wodurch die erste Streuinduktivität kleiner ist als die zweite Streuinduktivität. Durch die Verwendung von unterschiedlichen Durchmessern kann vorteilhafterweise eine Größe des Hochspannungstransformators verringert werden. Vorteilhafterweise kann weiterhin derart auf eine Verschaltung einer zusätzlichen Schwingkreisdrossel zur Einstellung der Induktivität mit dem Hochspannungstransformator verzichtet werden, wodurch Kosten und Bauraum eingespart werden. Vorteilhafterweise sind mittels dieser Ausführungsform die Streuinduktivitäten präzise einstellbar.

Eine Ausführungsform sieht vor, dass der Hochspannungstransformator einen Primärspulenkörper und einen Sekundärspulenkörper aufweist, wobei der Sekundärspulenkörper eine erste Sekundärspulenhälfte und eine zweite Sekundärspulenhälfte aufweist, wobei die erste Sekundärspulenhälfte die zumindest eine erste Sekundärwicklung aufweist, wobei die zweite Sekundärspulenhälfte die zumindest eine zweite Sekundärwicklung aufweist und wobei die erste Sekundärspulenhälfte und die zweite Sekundärspulenhälfte asymmetrisch zueinander aufgebaut sind. Vorzugsweise kann der Hochspannungstransformator die asymmetrische Last betreiben, ohne dass ein weiterer Hochspannungstransformator an die asymmetrische Last angeschlossen ist. In anderen Worten ist der Hochspannungstransformator ein asymmetrischer Hochspannungstransformator, welcher für eine asymmetrische Last, insbesondere die Röntgenröhre, ausgebildet ist. Da typischerweise lediglich der Hochspannungstransformator ohne den weiteren Hochspannungstransformator für den Betrieb der Röntgenröhre bereitgestellt wird, sinken die Kosten und der Bauraum. Vorteilhafterweise kann ein Grad einer Asymmetrie der asymmetrischen Last, insbesondere der Röntgenröhre, ermittelt werden, wobei der Hochspannungstransformator danach vorzugsweise gemäß dem Grad der Asymmetrie entworfen wird. In anderen Worten wird typischerweise in einer Laborumgebung der Grad der Asymmetrie ermittelt und/oder gemessen und daraufhin der Hochspannungstransformator an die asymmetrische Last derart angepasst, dass die Spannungsasymmetrie verringert ist.

Eine Ausführungsform sieht vor, dass die zumindest eine erste Sekundärwicklung in einer ersten Kammer der ersten Sekundärspulenhälfte gemeinsam mit zumindest einer ersten Papierisolationslage angeordnet ist, wobei die zumindest eine zweite Sekundärwicklung in einer zweiten Kammer der zweiten Sekundärspulenhälfte gemeinsam mit zumindest einer zweiten Papierisolationslage angeordnet ist und wobei sich eine Anordnung der ersten Papierisolationslage relativ zu der zumindest einen ersten Sekundärwicklung von einer Anordnung der zweiten Papierisolationslage relativ zu der zumindest einen zweiten Sekundärwicklung unterscheidet. Vorteilhafterweise kann jede Anordnung der Papierisolationslage einfach an die asymmetrische Last angepasst werden. Vorteilhafterweise sind mittels dieser Ausführungsform die Wicklungskapazitäten präzise einstellbar.

Eine Ausführungsform sieht vor, dass die zumindest eine erste Sekundärwicklung in einer ersten Kammer der ersten Sekundärspulenhälfte angeordnet ist, wobei die zumindest eine zweite Sekundärwicklung in einer zweiten Kammer der zweiten Sekundärspulenhälfte angeordnet ist und wobei eine erste Breite der ersten Kammer größer ist als eine zweite Breite der zweiten Kammer, wodurch die erste Wicklungskapazität größer ist als die zweite Wicklungskapazität. Diese Ausführungsform bietet insbesondere den Vorteil, dass diese bauliche Maßnahme, nämlich das Variieren der Breite der Kammern, vorzugsweise einen robusten Hochspannungstransformator ermöglicht. Vorteilhafterweise sind mittels dieser Ausführungsform die Wicklungskapazitäten präzise einstellbar.

Eine Ausführungsform sieht vor, dass der erste Durchmesser der zumindest einen ersten Sekundärwicklung kleiner ist als der zweite Durchmesser der zumindest einen zweiten Sekundärwicklung und dass die erste Breite der ersten Kammer größer ist als die zweite Breite der zweiten Kammer. Vorteilhafterweise stimmt die erste Resonanzfrequenz und die zweite Resonanzfrequenz überein, obwohl sich die erste Wicklungskapazität und die zweite Wicklungskapazität sowie die erste Streuinduktivität und die zweite Streuinduktivität betragsmäßig unterscheiden. Vorteilhafterweise sind mittels dieser Ausführungsform die Wicklungskapazitäten und die Streuinduktivitäten präzise einstellbar.

Die Schaltungsanordnung bietet insbesondere den Vorteil, dass der, insbesondere asymmetrische, Hochspannungstransformator, mit der, insbesondere als asymmetrische Last wirkende, Röntgenröhre elektrisch verbunden sein kann, wodurch der Betrieb der Röntgenröhre mittels des Hochspannungstransformators ermöglicht ist.

Eine Ausführungsform sieht vor, dass die Röntgenröhre ein Metallmittelteil aufweist und dass sich der Differenzstrom der Röntgenröhre zwischen der Anode und dem Metallmittelteil einstellt. Diese Ausführungsform bietet den Vorteil, dass die Spannungsasymmetrie am Hochspannungstransformator trotz der an der Anode zurückgestreuten Elektronen gering ausfällt.

Eine Ausführungsform sieht vor, dass zumindest eine Schwingkreisdrossel zwischen dem Hochspannungstransformator und der Röntgenröhre verschaltet ist. Diese Ausführungsform bietet insbesondere den Vorteil, dass der Hochspannungstransformator kleiner ausgebildet ist, wenn die zumindest eine Schwingkreisdrossel zusätzlich außerhalb des Hochspannungstransformators angeordnet ist.

Eine Ausführungsform sieht vor, dass zumindest ein Kondensator zwischen dem Hochspannungstransformator und der Röntgenröhre verschaltet ist. Diese Ausführungsform bietet insbesondere den Vorteil, dass der Hochspannungstransformator kleiner ausgebildet ist, wenn der zumindest eine Kondensator zusätzlich außerhalb des Hochspannungstransformators angeordnet ist.

Die erfindungsgemäße Röntgeneinrichtung weist eine Schaltungsanordnung und einen Röntgendetektor auf. Eine Fertigungszeit der Röntgeneinrichtung mit dem Hochspannungstransformator ist vorteilhafterweise geringer, weil typischerweise zur Reduzierung der Spannungsasymmetrie lediglich der Hochspannungstransformator gefertigt werden muss.

Die erfindungsgemäße Computertomograph weist die Röntgeneinrichtung auf. Der Computertomograph ist vorzugsweise kleiner im Vergleich zu einem herkömmlichen Computertomograph mit zwei herkömmlichen Hochspannungstransformatoren in Parallelschaltung. Typischerweise ist der Computertomograph günstiger herzustellen, weil die asymmetrische Last vorzugsweise mit dem asymmetrischen Hochspannungstransformator verbunden ist.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:

Fig. 1 ein Ersatzschaltbild eines Hochspannungstransformators in einem ersten Beispiel,

Fig. 2 einen schematischen Querschnitt durch einen Hochspannungstransformator in einem zweiten Beispiel,

Fig. 3 einen schematischen asymmetrischen Aufbau eines Hochspannungstransformator in einem dritten Beispiel,

Fig. 4 einen schematischen Querschnitt durch einen Hochspannungstransformator in einem vierten Beispiel,

Fig. 5 ein Blockschaltbild einer Schaltungsanordnung gemäß einem Ausführungsbeispiel und

Fig. 6 eine Röntgeneinrichtung in einem weiteren Ausführungsbeispiel.

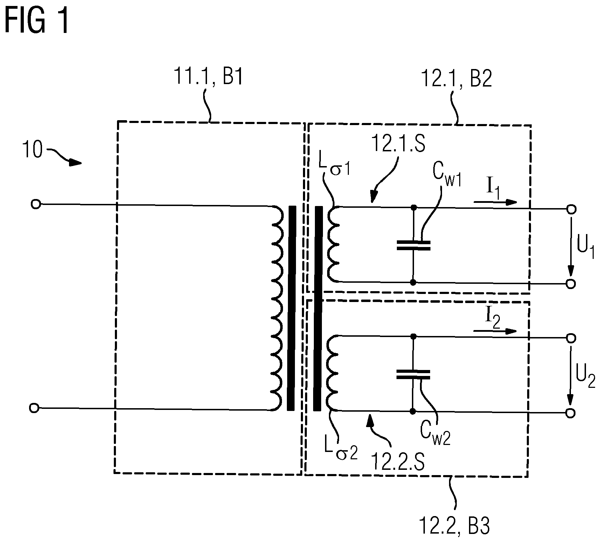

**Fig. 1** zeigt einen Hochspannungstransformator 10 für eine Röntgenröhre 20, welche in Fig. 1 nicht gezeigt ist, in einem Ersatzschaltbild. Der Hochspannungstransformator 10 weist zumindest eine Primärwicklung 11.1, zumindest eine erste Sekundärwicklung 12.1 und zumindest eine zweite Sekundärwicklung 12.2 auf. Der Hochspannungstransformator 10 kann weitere Elemente aufweisen, welche in Fig. 1 nicht gezeigt sind.

Der Hochspannungstransformator 10 ist in dem Ersatzschaltbild als realer Hochspannungstransformator abgebildet. Der Hochspannungstransformator 10 ist typischerweise ein asymmetrischer Hochspannungstransformator.

Das Ersatzschaltbild des Hochspannungstransformators 10 ist in Fig. 1 in drei Bereiche B1, B2, B3 aufgeteilt, wobei die drei Bereiche B1, B2, B3 jeweils mit gestrichelten Linien umrandet sind. In den drei Bereichen B1, B2, B3 sind Ersatzschaltungen der zumindest eine Primärwicklung 11.1, der zumindest eine erste Sekundärwicklung 12.1 und der zumindest eine zweite Sekundärwicklung 12.2 abgebildet.

Die zumindest eine erste Sekundärwicklung 12.1 weist einen ersten Schwingkreis 12.1.S auf, wobei der erste Schwingkreis 12.1.S eine erste Streuinduktivität $L_{\sigma 1}$ und eine erste Wicklungskapazität $C_{W1}$ aufweist, welche gemeinsam eine erste Resonanzfrequenz einstellen.

Die zumindest eine zweite Sekundärwicklung 12.2 weist einen zweiten Schwingkreis 12.2.S auf, wobei der zweite Schwingkreis 12.2.S eine zweite Streuinduktivität $L_{\sigma 2}$ und eine zweite Wicklungskapazität $C_{W2}$ aufweist, welche gemeinsam eine zweite Resonanzfrequenz einstellen.

Die erste Resonanzfrequenz und die zweite Resonanzfrequenz stimmen überein. In anderen Worten entsprechen sich die erste Resonanzfrequenz und die zweite Resonanzfrequenz betragsmäßig. Die erste Wicklungskapazität $C_{W1}$ sowie die zweite Wicklungskapazität $C_{W2}$ unterscheiden sich betragsmäßig und die erste Streuinduktivität $L_{\sigma 1}$ sowie die zweite Streuinduktivität $L_{\sigma 2}$ unterscheiden sich betragsmäßig.

In der zumindest einen ersten Sekundärwicklung 12.1 stellt sich ein erster Laststrom I1 ein und in der zumindest einen zweiten Sekundärwicklung 12.1 stellt sich ein zweiter Laststrom I2 ein, insbesondere wenn der Hochspannungstransformator 10 mit einer asymmetrischen Last verbunden ist. In anderen Worten fließt der erste Laststrom I1 in der ersten Sekundärwicklung 12.1 und der zweite Laststrom I2 in der zweiten Sekundärwicklung 12.2. Das Einstellen des ersten Laststroms I1 und/oder des zweiten Laststroms I2 bedeutet typischerweise, dass der Hochspannungstransformator 10 durch ein Betreiben einer Last, insbesondere der asymmetrischen Last, belastet wird. Wenn sich der erste Laststrom I1 einstellt, fällt typischerweise eine erste Hochspannung U1 über der zumindest einen ersten Sekundärwicklung 12.1 ab. Wenn sich der zweite Laststrom I2 einstellt, fällt typischerweise eine zweite Hochspannung U2 über der zumindest einen zweiten Sekundärwicklung 12.2 ab. Die erste Hochspannung U1 und die zweite Hochspannung U2 sind typischerweise Wechselspannungen.

Wenn die Last die asymmetrische Last, beispielsweise die Röntgenröhre 20, ist, stimmen vorzugsweise die erste Hochspannung U1 mit der zweiten Hochspannung U2 betragsmäßig überein, wobei sich der erste Laststrom I1 von dem zweiten Laststrom I2 betragsmäßig unterscheidet. In diesem Fall ist vorzugsweise eine Spannungsasymmetrie am Hochspannungstransformator 10 dadurch geringer, besonders vorzugsweise vermieden, weil die erste Resonanzfrequenz und die zweite Resonanzfrequenz übereinstimmen, sich die erste Wicklungskapazität $C_{W1}$ und die zweite Wicklungskapazität $C_{W2}$ betragsmäßig unterscheiden und weil sich die erste Streuinduktivität $L_{\sigma1}$ und die zweite Streuinduktivität $L_{\sigma2}$ betragsmäßig unterscheiden. In anderen Worten ist der Hochspannungstransformator 10 vorzugsweise der asymmetrische Hochspannungstransformator, wobei im Betrieb mit der asymmetrischen Last, insbesondere mit der Röntgenröhre 20, die Spannungsasymmetrie geringer ist im Vergleich dazu, wenn der Hochspannungstransformator 10 ein herkömmlicher symmetrischer Hochspannungstransformator ist.

Der erste Laststrom I1 unterscheidet sich von dem zweiten Laststrom I2, insbesondere betragsmäßig, um einen Differenzstrom 10.I∆ des Hochspannungstransformators 10. Der Differenzstrom 10.I∆ des Hochspannungstransformators 10 ist typischerweise ein Wechselstrom.

Der Differenzstrom 10.I∆ des Hochspannungstransformators 10 ist proportional zu einem Differenzstrom 20.I∆ der Röntgenröhre 20, welcher typischerweise ein Gleichstrom ist. In anderen Worten korreliert der Differenzstrom 20.I∆ der Röntgenröhre 20 mit dem Differenzstrom 10.I∆ des Hochspannungstransformators 10. Ein Gleichrichtmittelwert des Differenzstroms 10.I∆ des Hochspannungstransformators 10 und ein Betrag des Differenzstroms 20.I∆ der Röntgenröhre 20 unterscheiden sich beispielsweise um einen Faktor 0 bis 10, besonders vorteilhafterweise um einen Faktor 2 bis 3.

Der Differenzstrom 10.I∆ des Hochspannungstransformators 10 wird typischerweise dadurch erzeugt, dass sich die erste Wicklungskapazität $C_{W1}$ und die zweite Wicklungskapazität $C_{W2}$ sowie die erste Streuinduktivität $L_{\sigma1}$ und die zweite Streuinduktivität $L_{\sigma2}$ betragsmäßig unterscheiden, wobei die erste Resonanzfrequenz und die zweite Resonanzfrequenz übereinstimmen. Typischerweise ist der erste Laststrom I1 desto höher, je kleiner die erste Streuinduktivität $L_{\sigma1}$ ist. Alternativ oder zusätzlich ist der erste Laststrom I1 desto höher, je größer die erste Wicklungskapazität $C_{W1}$ ist, insbesondere wenn die erste Resonanzfrequenz und die zweite Resonanzfrequenz übereinstimmen.

In einem Ausführungsbeispiel stellt sich der Differenzstrom 20.I∆ der Röntgenröhre 20 in der Röntgenröhre 20 ein. In anderen Worten fließt der Differenzstrom 20.I∆ der Röntgenröhre 20 vorzugsweise in der Röntgenröhre 20 ab. Besonders vorteilhafterweise entsprechen die an einer Anode 22 der Röntgenröhre 20 zurückgestreuten Elektronen betragsmäßig dem Differenzstrom 20.I∆ der Röntgenröhre 20. In anderen Worten weist der Differenzstrom 20.I∆ der Röntgenröhre 20 vorzugsweise die an der Anode 22 der Röntgenröhre 20 zurückgestreuten Elektronen auf. Das Zurückstreuen der Elektronen an der Anode 22 kann ein Abprallen an der Anode 22 umfassen. Der Differenzstrom 20.I∆ der Röntgenröhre 20 kann weitere Elektronen umfassen, welche vorher nicht an der Anode 22 aufgetroffen sind, sondern typischerweise direkt von der Kathode 21 zum Metallmittelteil 23 gestreut werden. Der Differenzstrom 20.I∆ der Röntgenröhre 20 umfasst typischerweise weniger als 20%, insbesondere weniger als 10%, aller von der Kathode 21 emittierten Elektronen.

Der Differenzstrom 20.I∆ der Röntgenröhre 20 kann als Verluststrom und/oder parasitärer Strom bezeichnet werden. Grundsätzlich ist es denkbar, dass eine Anzahl von an der Anode 22 der Röntgenröhre 20 zurückgestreuten Elektronen derart variiert, dass der Hochspannungstransformator 10, insbesondere der asymmetrische Hochspannungstransformator, eine geringe Spannungsasymmetrie aufweist.

Es ist allgemein bekannt, dass im Hinblick auf technische Eigenschaften die zumindest eine Primärwicklung 11.1, die zumindest eine erste Sekundärwicklung 12.1 und die zumindest eine zweite Sekundärwicklung 12.2 üblicherweise vergleichbar sind. Daher werden im Folgenden, um unnötige Wiederholungen zu vermeiden, die technischen Eigenschaften vorzugsweise in Bezug auf die zumindest eine erste Sekundärwicklung 12.1 beschrieben, wobei diese Beschreibung entsprechend auf die zumindest eine Primärwicklung 11.1 und/oder die zumindest eine zweite Sekundärwicklung 12.1 übertragen werden kann.

Wenn die zumindest eine erste Sekundärwicklung 12.1 den ersten Schwingkreis 12.1.S aufweist, entspricht ein Übertragungsverhalten des Hochspannungstransformators 10 üblicherweise einem Übertragungsverhalten des realen Hochspannungstransformators. Die zumindest eine erste Sekundärwicklung 12.1 weist den ersten Schwingkreis 12.1.S typischerweise dadurch auf, dass die zumindest eine erste Sekundärwicklung 12.1 sich insbesondere wie eine elektrische Schaltung mit der ersten Streuinduktivität $L_{\sigma1}$ und mit der ersten Wicklungskapazität $C_{W1}$ verhält. In anderen Worten erzeugen die erste Streuinduktivität $L_{\sigma1}$ und die erste Wicklungskapazität $C_{W1}$ üblicherweise den ersten Schwingkreis 12.1.S der zumindest einen ersten Sekundärwicklung 12.1.

Die Bezugszeichen $C_{W1}$, $C_{W2}$, $L_{\sigma1}$, $L_{\sigma2}$ sind ebenfalls Formelzeichen $C_{W1}$, $C_{W2}$, $L_{\sigma1}$, $L_{\sigma2}$ der entsprechenden Streuinduktivitäten und Wicklungskapazitäten.

Die erste Resonanzfrequenz berechnet sich wie folgt:

```
f₁ = 1 / (2 * π * (Lσ1 * Cw1)^(1/2))
```

Wenn die erste Resonanzfrequenz mit der zweiten Resonanzfrequenz übereinstimmt, ist vorzugsweise folgende Gleichung erfüllt:

$$L_{\sigma 1} \; * \; C_{W1} \; = \; L_{\sigma 2} \; * \; C_{W2}$$

Wenn sich die erste Wicklungskapazität $C_{W1}$ betragsmäßig von der zweiten Wicklungskapazität $C_{W2}$ unterscheidet, ist vorzugsweise folgende Gleichung erfüllt:

$$C_{W1} \; != \; C_{W2}$$

Wenn sich die erste Streuinduktivität $L_{\sigma 1}$ betragsmäßig von der zweiten Streuinduktivität $L_{\sigma 2}$ unterscheidet, ist vorzugsweise folgende Gleichung erfüllt:

$$L_{\sigma 1} \; != \; L_{\sigma 2}$$

Betragsmäßig übereinstimmen oder betragsmäßig entsprechen bedeutet insbesondere, dass ein erster Wert, welcher eine erste nichtnegative reelle Zahl ist, einem zweiten Wert, welcher eine zweite nichtnegative reelle Zahl ist, gleicht. In anderen Worten gleichen sich beide Werte. Wenn beispielsweise die erste Resonanzfrequenz als erster Wert 100 kHz und die zweite Resonanzfrequenz als zweiter Wert 100 kHz beträgt, dann stimmen der erste Wert und der zweite Wert überein.

Betragsmäßig unterscheiden bedeutet insbesondere, dass der erste Wert größer oder kleiner ist als der zweite Wert. Wenn beispielsweise die erste Wicklungskapazität $C_{W1}$ als erster Wert 200 nF und die zweite Wicklungskapazität $C_{W2}$ als zweiter Wert 100 nF beträgt, dann unterscheiden sich der erste Wert und der zweite Wert, weil der zweite Wert größer ist als der erste Wert.

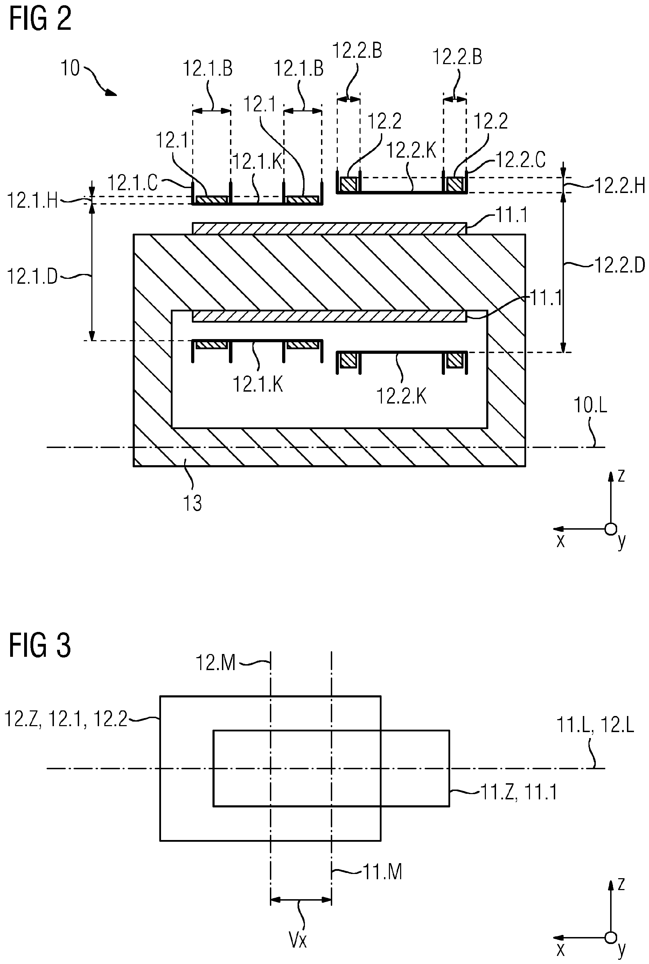

**Fig. 2** zeigt den Hochspannungstransformator 10 in einem schematischen Querschnitt. Der Hochspannungstransformator 10 weist einen Transformatorkern 13 auf, wobei die zumindest eine Primärwicklung 11.1, die zumindest eine erste Sekundärwicklung 12.1 und die zumindest eine zweite Sekundärwicklung 12.2 gemäß einer Mantelwicklung um den Transformatorkern 13 gewickelt sind. Typischerweise sind gemäß der Mantelwicklung die zumindest eine Primärwicklung 11.1, die zumindest eine erste Sekundärwicklung 12.1 und die zumindest eine zweite Sekundärwicklung 12.2 um denselben Schenkel des Transformatorkerns 13 gewickelt. Alternativ ist es denkbar, dass die zumindest eine Primärwicklung 11.1, die zumindest eine erste Sekundärwicklung 12.1 und die zumindest eine zweite Sekundärwicklung 12.2 um zwei oder drei verschiedene Schenkel des Transformatorkerns 13 gewickelt sind. Der Transformatorkern 13 weist üblicherweise einen Magnetkern aus einem weichmagnetischen Werkstoff mit hoher magnetischer Permeabilität auf.

Der Hochspannungstransformator 10 weist einen Primärspulenkörper und einen Sekundärspulenkörper auf. Der Primärspulenkörper und/oder der Sekundärspulenkörper sind typischerweise nicht-metallisch, insbesondere aus Plastik, ausgebildet. Typischerweise wirken der Primärspulenkörper und der Sekundärspulenkörper als Isolation zwischen den Wicklungen 11.1, 12.1, 12.2 und dem Transformatorkern 13. Vorzugsweise haben der Primärspulenkörper und/oder der Sekundärspulenkörper geringen, besonders vorteilhafterweise keinen, Einfluss auf die Streuinduktivitäten $L_{\sigma 1}$, $L_{\sigma 2}$ und die Wicklungskapazitäten $C_{W1}$, $C_{W2}$. Der Primärspulenkörper sowie weitere typische Bauelemente des Hochspannungstransformators 10 sind in Fig. 2 aus Gründen der Übersichtlichkeit nicht gezeigt.

Der Sekundärspulenkörper weist eine erste Sekundärspulenhälfte 12.1.K und eine zweite Sekundärspulenhälfte 12.2.K auf. Die erste Sekundärspulenhälfte 12.1.K und die zweite Sekundärspulenhälfte 12.2.K können als Teil eines einzigen Sekundärspulenkörpers ausgebildet sein oder als zwei voneinander getrennte Teile des Sekundärspulenkörpers, wie in Fig. 2 gezeigt, ausgebildet sein. In anderen Worten kann die erste Sekundärspulenhälfte 12.1.K von der zweiten Sekundärspulenhälfte 12.2.K baulich getrennt sein.

Die erste Sekundärspulenhälfte 12.1.K weist die zumindest eine erste Sekundärwicklung 12.1 auf und die zweite Sekundärspulenhälfte 12.2.K weist die zumindest eine zweite Sekundärwicklung 12.2 auf. Der Primärspulenkörper und der Sekundärspulenkörper, insbesondere die erste Sekundärspulenhälfte 12.1.K und die zweite Sekundärspulenhälfte 12.2.K, wirken typischerweise als eine Haltevorrichtung für die jeweils angeordneten Wicklungen 11.1, 12.1, 12.2. Beispielsweise wird die zumindest eine erste Sekundärwicklung 12.1 mittels der ersten Sekundärspulenhälfte 12.1.K mechanisch getragen, während die zumindest eine zweite Sekundärwicklung 12.2 mittels der zweiten Sekundärspulenhälfte 12.2.K mechanisch getragen wird. In anderen Worten ist die zumindest eine erste Sekundärwicklung 12.1 um die erste Sekundärspulenhälfte 12.1.K sowie die zumindest eine zweite Sekundärwicklung 12.2 um die zweite Sekundärspulenhälfte 12.2.K angeordnet und/oder gewickelt.

Die erste Sekundärspulenhälfte 12.1.K und die zweite Sekundärspulenhälfte 12.2.K sind asymmetrisch zueinander aufgebaut. Der asymmetrische Aufbau kann aus einer mechanischen Maßnahme erfolgen und/oder gemäß einer Auswahl unterschiedlicher Materialen für die Ausbildung der Wicklungen 12.1, 12.2 erwirkt sein. Die Materialien unterscheiden sich typischerweise in einer Materialbeschaffenheit, wie beispielsweise in einer elektrischen und/oder magnetischen Leitfähigkeit bzw. Kapazität.

Es ist allgemein bekannt, dass der asymmetrische Aufbau mehrere technische Effekte aufweisen kann, beispielsweise:

- eine Variation der ersten Wicklungskapazität $C_{W1}$,
- eine Variation der zweiten Wicklungskapazität $C_{W2}$,
- eine Variation der erste Streuinduktivität $L_{\sigma1}$,
- eine Variation der zweiten Streuinduktivität $L_{\sigma2}$ und/oder
- eine beliebige Kombination der voranstehenden technischen Effekte. Alternativ oder zusätzlich ist es denkbar, dass trotz mehrerer technischer Effekte ein erster technischer Effekt überwiegt und/oder ein zweiter technischer Effekt, insbesondere in einer idealen Umgebung, vernachlässigbar ist.

Weiterhin ist es denkbar, dass verschiedene Maßnahmen, insbesondere eine der mechanischen Maßnahmen mit der Auswahl der unterschiedlichen Materialien für die Ausbildung der Wicklungen 12.1, 12.2 beliebig derart kombiniert werden, dass die erste Resonanzfrequenz und die zweite Resonanzfrequenz übereinstimmen, wobei sich die erste Wicklungskapazität $C_{W1}$ und die zweite Wicklungskapazität $C_{W2}$ betragsmäßig unterscheiden und wobei sich die erste Streuinduktivität $L_{\sigma1}$ und die zweite Streuinduktivität $L_{\sigma2}$ betragsmäßig unterscheiden.

Typischerweise unterscheidet sich aufgrund der Asymmetrie der ersten Sekundärspulenhälfte 12.1.K und der zweiten Sekundärspulenhälfte 12.2.K insbesondere eine Raumlage der zumindest einen ersten Sekundärwicklung 12.1 von einer Raumlage der zumindest einen zweiten Sekundärwicklung 12.2, typischerweise aufgrund der mechanischen Maßnahme. Die Raumlage der zumindest einen ersten Sekundärwicklung 12.1 weist beispielsweise eine Position, eine Orientierung, eine Wicklungshöhe und/oder einen Durchmesser auf. Beispielsweise beeinflusst die Raumlage der zumindest einen ersten Sekundärwicklung 12.1 relativ zum Transformatorkern 13 einen Streufluss, wobei der Streufluss typischerweise mit der ersten Streuinduktivität $L_{\sigma1}$ korreliert. Alternativ oder zusätzlich variiert typischerweise die erste Wicklungskapazität $C_{W1}$, wenn die Raumlage der zumindest einen ersten Sekundärwicklung 12.1 in Bezug auf sich selbst verändert wird, insbesondere wenn die Wicklungshöhe der zumindest einen ersten Sekundärwicklung 12.1 variiert wird.

In diesem Beispiel ist ein erster Durchmesser 12.1.D der zumindest einen ersten Sekundärwicklung 12.1 kleiner als ein zweiter Durchmesser 12.2.D der zumindest einen zweiten Sekundärwicklung 12.2, wodurch die erste Streuinduktivität $L_{\sigma1}$ kleiner ist als die zweite Streuinduktivität $L_{\sigma2}$. In diesem Fall ist ein Streufluss durch die zumindest eine erste Sekundärwicklung 12.1, insbesondere die Verluste, kleiner im Vergleich zu einem Streufluss durch die zumindest eine zweite Sekundärwicklung 12.2. Wenn der erste Durchmesser 12.1.D sich von dem zweiten Durchmesser 12.2.D unterscheidet, ist beispielsweise die Raumlage der zumindest einen ersten Sekundärwicklung 12.1 in Bezug auf die Raumlage der zumindest einen zweiten Sekundärwicklung 12.2 verschieden.

In diesem Beispiel ist die zumindest eine erste Sekundärwicklung 12.1 in einer ersten Kammer 12.1.C der ersten Sekundärspulenhälfte 12.1.K angeordnet ist, wobei die zumindest eine zweite Sekundärwicklung 12.2 in einer zweiten Kammer 12.2.C der zweiten Sekundärspulenhälfte 12.2.K angeordnet ist und wobei eine erste Breite 12.1.B der ersten Kammer 12.1.C größer ist als eine zweite Breite 12.2.B der zweiten Kammer 12.2.C, wodurch die erste Wicklungskapazität $C_{W1}$ größer ist als die zweite Wicklungskapazität $C_{W2}$. Die erste Kammer 12.1.C und die zweite Kammer 12.2.C wirken typischerweise derart, dass die zumindest eine erste Sekundärwicklung 12.1 relativ zur ersten Sekundärspulenhälfte 12.1.K sowie die zumindest eine zweite Sekundärwicklung 12.2 relativ zur zweiten Sekundärspulenhälfte 12.2.K mechanisch gehalten und/oder fixiert sind. Jede Kammer 12.1.C, 12.2.C weist üblicherweise Stege auf, wobei die Stege gemeinsam mit dem Sekundärspulenkörper ein u-förmiges Profil der Kammern 12.1.C, 12.2.C bewirken. Alternativ oder zusätzlich können die Kammern 12.1.C, 12.2.C ein o-förmiges Profil und/oder ohne die Stege gar kein Profil aufweisen.

Der Hochspannungstransformator 10 weist zumindest eine Längsachse 10.L auf, wobei die Längsachse 10.L in diesem Beispiel parallel zur x-Achse ausgebildet ist. Die erste Breite 12.1.B und die zweite Breite 12.2.B beschreiben in diesem Beispiel insbesondere eine Breite der Kammern 12.1.C, 12.2.C entlang der x-Achse. Der erste Durchmesser 12.1.D und der zweite Durchmesser 12.2.D sind in diesem Beispiel parallel zur z-Achse.

Typischerweise weisen die zumindest eine erste Sekundärwicklung 12.1 und die zumindest eine zweite Sekundärwicklung 12.2 eine gleiche Anzahl an Wicklungsdrähten auf. Insbesondere weisen die zumindest eine erste Sekundärwicklung 12.1 und die zumindest eine zweite Sekundärwicklung 12.2 mehrere Wicklungsdrähte auf. Die Wicklungsdrähte sind typischerweise aus Kupfer.

Wenn die erste Breite 12.1.B größer ist als die zweite Breite 12.2.B, ist üblicherweise eine erste Wicklungshöhe 12.1.H der zumindest einen ersten Sekundärwicklung 12.1 geringer als eine zweite Wicklungshöhe 12.2.H der zumindest einen zweiten Sekundärwicklung 12.2. Wenn die erste Wicklungshöhe 12.1.H der zumindest einen ersten Sekundärwicklung 12.1 geringer ist als die zweite Wicklungshöhe 12.2.H der zumindest einen zweiten Sekundärwicklung 12.2, ist üblicherweise die erste Streuinduktivität $L_{\sigma1}$ kleiner als die zweite Streuinduktivität $L_{\sigma2}$. Üblicherweise unterscheiden sich daher die erste Streuinduktivität $L_{\sigma1}$ und die zweite Streuinduktivität $L_{\sigma2}$, wenn die erste Breite 12.1.B sich von der zweiten Breite 12.2.B unterscheidet. Eine Wicklungsbreite ist typischerweise indirekt proportional zur Wicklungshöhe 12.1.H, 12.2.H.

Ein besonders vorteilhaftes Beispiel kann sein, wenn sich die erste Wicklungskapazität $C_{W2}$ und die zweite Wicklungskapazität $C_{W2}$ sowie die erste Streuinduktivität $L_{\sigma1}$ und die zweite Streuinduktivität $L_{\sigma2}$ betragsmäßig dadurch unterscheiden, weil die erste Breite 12.1.B größer ist als die zweite Breite 12.2.B und weil der erste Durchmesser 12.1.D kleiner ist als der zweite Durchmesser 12.2.D.

In einem weiteren Beispiel werden unterschiedliche Materialen für die Ausbildung der Wicklungen 12.1, 12.2 gewählt. Ein Wicklungsdraht der zumindest einen ersten Sekundärwicklung 12.1 unterscheidet sich von einem Wicklungsdraht der zumindest einen zweiten Sekundärwicklung 12.1 in diesem Fall gemäß zumindest einem Parameter der folgenden Liste:

- einer Dicke des Wicklungsdrahts,
- einer Dicke einer Lackisolationsschicht des Wicklungsdrahts,
- einer Dielektrizitätszahl der Lackisolationsschicht des Wicklungsdrahts. In diesem Fall bewirkt die Auswahl der unterschiedlichen Materialen, dass die erste Wicklungskapazität $C_{W1}$ sowie die zweite Wicklungskapazität $C_{W2}$ sich unterscheiden.

**Fig. 3** zeigt den Hochspannungstransformator 10, insbesondere eine Raumlage der zumindest einen Primärwicklung 11.1, eine Raumlage der zumindest einen ersten Sekundärwicklung 12.1 und eine Raumlage der zumindest einen zweiten Sekundärwicklung 12.2 relativ zueinander, in einem Schema gemäß einem asymmetrischen Aufbau.

Die zumindest eine Primärwicklung 11.1 bildet einen ersten Zylinder 11.Z mit einer ersten Längsachse 11.L, wobei die zumindest eine erste Sekundärwicklung 12.1 und die zumindest eine zweite Sekundärwicklung 12.2 gemeinsam einen zweiten Zylinder 12.Z mit einer zweiten Längsachse 12.L bilden. Das imaginäre Bilden der Zylinder dient insbesondere dem Zweck, die Position relativ zueinander darzustellen. Grundsätzlich können die zumindest eine Primärwicklung 11.1, die zumindest eine erste Sekundärwicklung 12.1 und/oder die zumindest eine zweite Sekundärwicklung 12.2 zylinderförmig ausgebildet sein.

Die erste Längsachse 11.L ist parallel zur zweiten Längsachse 12.L sowie zu der in Fig. 3 gezeigten x-Achse ausgebildet. In diesem Beispiel ist die erste Längsachse 11.L deckungsgleich zur zweiten Längsachse 12.L.

Der erste Zylinder 11.Z weist eine erste Mittellinie 11.M auf, wobei der zweite Zylinder 12.Z eine zweite Mittellinie 12.M aufweist. Die erste Mittellinie 11.M und die zweite Mittellinie 12.M kennzeichnen jeweils eine Mitte des ersten Zylinders 11.Z bzw. des zweiten Zylinders 12.Z.

Der erste Zylinder 11.Z ist relativ zum zweiten Zylinder 12.Z entlang der ersten Längsachse 11.L, insbesondere um den Versatz Vx, versetzt angeordnet. In anderen Worten ist die erste Mittellinie 11.M entlang der ersten Längsachse 11.L, insbesondere entlang der x-Achse, um den Versatz Vx relativ zur zweiten Mittellinie 12.M verschoben. Grundsätzlich ist es denkbar, dass der erste Zylinder 11.Z relativ zum zweiten Zylinder entlang der z-Achse und/oder entlang der y-Achse verschoben ist. In anderen Worten kann der erste Zylinder 11.Z relativ zum zweiten Zylinder 12.Z um einen Versatz in jede der Raumrichtungen versetzt angeordnet sein, wodurch der erste Zylinder 11.Z relativ zum zweiten Zylinder 12.Z verdreht angeordnet sein kann. Versetzt angeordnet kann bedeuten, dass der erste Zylinder 11.Z relativ zum zweiten Zylinder 12.Z aus einer gemeinsamen Mitte heraus verschoben ist.

Wenn der erste Zylinder 11.Z zum zweiten Zylinder 12.Z versetzt angeordnet ist, unterscheiden sich typischerweise die erste Streuinduktivität $L_{\sigma1}$ von der zweiten Streuinduktivität $L_{\sigma2}$ betragsmäßig.

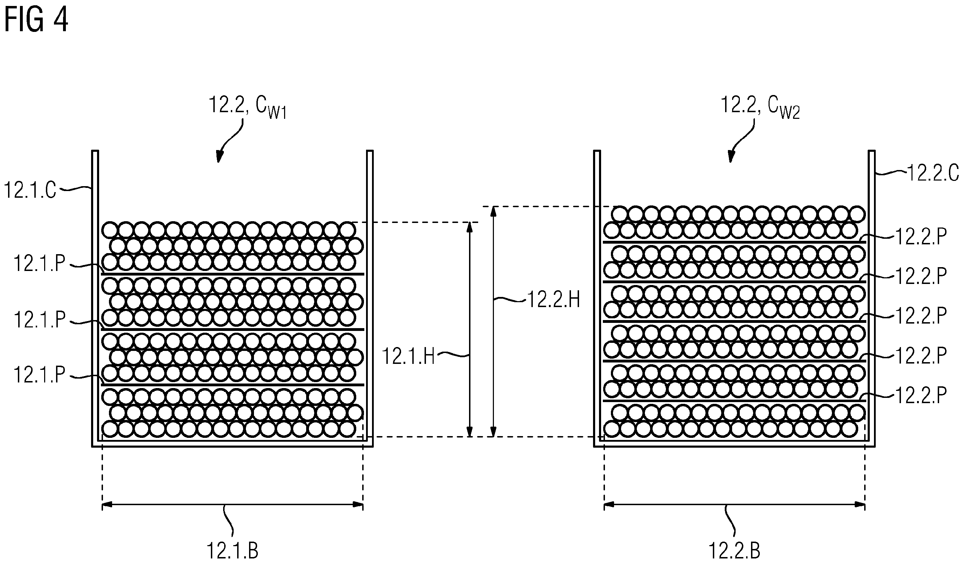

**Fig. 4** zeigt den Hochspannungstransformator 10, insbesondere die erste Kammer 12.1.C und die zweite Kammer 12.2.C, in einem schematischen Querschnitt.

Die zumindest eine erste Sekundärwicklung 12.1 ist in der ersten Kammer 12.1.C der ersten Sekundärspulenhälfte 12.1.K gemeinsam mit zumindest einer ersten Papierisolationslage 12.1.P angeordnet.

Die zumindest eine zweite Sekundärwicklung 12.2 ist in der zweiten Kammer 12.2.C der zweiten Sekundärspulenhälfte 12.2.K gemeinsam mit zumindest einer zweiten Papierisolationslage 12.2.P angeordnet.

Die erste Kammer 12.1.C und die zweite Kammer 12.2.C weisen das u-förmige Profil auf. Im Gegensatz zum dem in Fig. 2 gezeigten Beispiel ist die erste Breite 12.1.B der ersten Kammer 12.1.C betragsmäßig gleich zur zweiten Breite 12.2.B der zweiten Kammer 12.2.C.

Eine Anordnung der ersten Papierisolationslage 12.1.P relativ zu der zumindest einen ersten Sekundärwicklung 12.1 unterscheidet sich von einer Anordnung der zweiten Papierisolationslage 12.2.P relativ zu der zumindest einen zweiten Sekundärwicklung 12.2. Die zumindest eine erste Sekundärwicklung 12.1 und die zumindest eine zweite Sekundärwicklung 12.2 weisen die gleiche Anzahl an Wicklungsdrähten auf, wobei die Wicklungsdrähte in diesem Beispiel jeweils in 12 Wicklungslagen in der ersten Kammer 12.1.C und in der zweiten Kammer 12.2.C angeordnet sind. Die erste Papierisolationslage 12.1.P umfasst beispielsweise drei Trennschichten, während die zweite Papierisolationslage 12.2.P fünf Trennschichten umfasst, wodurch die erste Wicklungshöhe 12.1.H kleiner ist als die zweite Wicklungshöhe 12.2.H. Die erste Wicklungskapazität $C_{W1}$ ist in diesem Beispiel größer als die zweite Wicklungskapazität $C_{W2}$. Typischerweise sind die Wicklungskapazitäten $C_{W1}$, $C_{W2}$ desto größer, je mehr Wicklungslagen pro Trennschicht angeordnet sind.

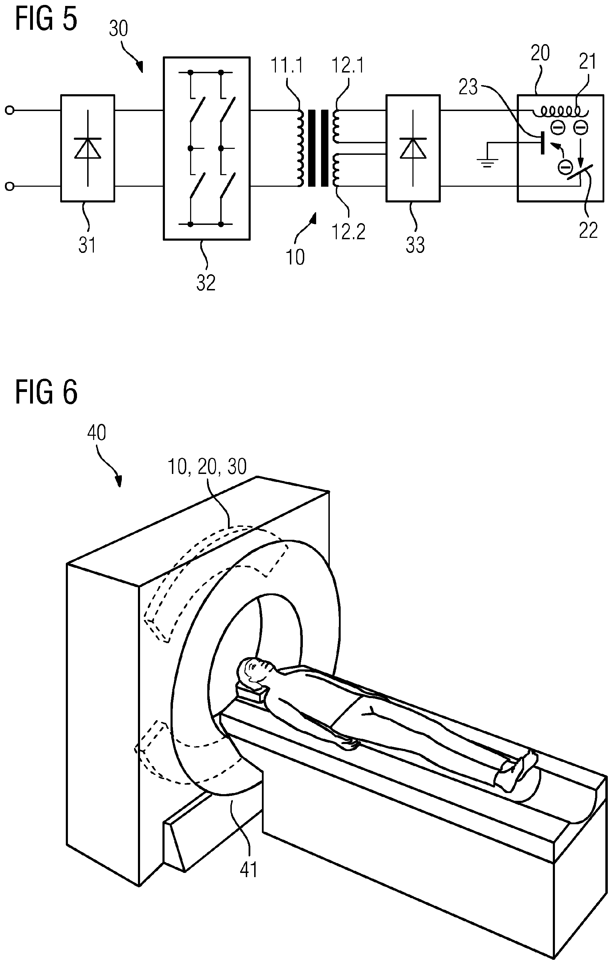

**Fig. 5** zeigt eine Schaltungsanordnung 30 in einem Blockschaltbild. Die Schaltungsanordnung 30 weist den Hochspannungstransformator 10 und die Röntgenröhre 20 auf. Die Röntgenröhre 20 weist eine Kathode 21 und eine Anode 22 auf. Die zumindest eine erste Sekundärwicklung 12.1 ist mit der Kathode 21 elektrisch verbunden und die zumindest eine zweite Sekundärwicklung 12.2 ist mit der Anode 22 elektrisch verbunden. Auf einer Eingangsseite des Hochspannungstransformators 10 sind eine Netzeingangsschaltung 31 und ein Wechselrichterschaltung 32 verschaltet. Die Wechselrichterschaltung 32 kann eine Halbbrücken- und/oder eine Vollbrückenschaltung aufweisen. Zwischen dem Hochspannungstransformator 10 und der Röntgenröhre 20 ist auf einer Ausgangsseite ein Gleichrichter 33 verschaltet. Der Gleichrichter 33 kann ein Spannungsverdopplergleichrichter sein. Typischerweise weist der Gleichrichter 33 auf einer ersten Seite den Differenzstrom 10.I$\Delta$ des Hochspannungstransformators 10 und/oder auf einer zweiten Seite den Differenzstrom 20.I$\Delta$ der Röntgenröhre 20 auf.

In diesem Ausführungsbeispiel weist die Röntgenröhre 20 ein Metallmittelteil 23 auf. Der Differenzstrom 20.I$\Delta$ der Röntgenröhre 20 stellt sich zwischen der Anode 22 und dem Metallmittelteil 23 ein. In diesem Fall ist das Metallmittelteil 23 typischerweise geerdet, worüber der Differenzstrom 20.I$\Delta$ der Röntgenröhre 20 abfließt.

Grundsätzlich ist es denkbar, zumindest eine Schwingkreisdrossel zwischen dem Hochspannungstransformator 10 und der Röntgenröhre 20 verschaltet ist. Vorteilhafterweise kann mittels der zumindest einen Schwingkreisdrossel die erste Streuinduktivität $L_{\sigma 1}$ und/oder die zweite Streuinduktivität $L_{\sigma 2}$ beeinflusst und/oder eingestellt sein.

In einem weiteren Ausführungsbeispiel ist zumindest ein Kondensator zwischen dem Hochspannungstransformator 10 und der Röntgenröhre 20, insbesondere parallel zur zumindest einen ersten Sekundärwicklung 12.1 und/oder parallel zur zumindest einen zweiten Sekundärwicklung 12.2, verschaltet. Vorteilhafterweise kann mittels des zumindest einen Kondensators die erste Wicklungskapazität $C_{W1}$ und/oder die zweite Wicklungskapazität $C_{W2}$ beeinflusst und/oder eingestellt sein.

**Fig. 6** zeigt eine Röntgeneinrichtung 40. Die Röntgeneinrichtung 40 weist die Schaltungsanordnung 30 auf. Die Röntgeneinrichtung 40 weist zusätzlich zur Schaltungsanordnung 30 einen Röntgendetektor 41 auf, welcher eine an der Anode 22 erzeugte Röntgenstrahlung erfassen kann. Die Röntgeneinrichtung 40 kann beispielsweise für eine medizinische Bildgebung, insbesondere eine Angiographie-Untersuchung, verwendet werden. Grundsätzlich ist es denkbar, dass mittels der Röntgeneinrichtung 40 nicht-medizinische Bildgebung durchgeführt wird.

In diesem Ausführungsbeispiel ist die Röntgeneinrichtung 40 als ein Computertomograph ausgebildet. Der Computertomograph weist eine Patientenliege auf, um welche typischerweise die Röntgenröhre 20 und der Röntgendetektor 41 drehbar gelagert sind. Grundsätzlich ist es denkbar, dass der Hochspannungstransformator 10 derart aufgebaut ist, dass die zumindest eine Primärwicklung 11.1 stationär ist, während die zumindest eine erste Sekundärwicklung 12.1 und die zumindest eine zweite Sekundärwicklung 12.2 sich mit der Röntgenröhre 20 und dem Röntgendetektor 41 um die Patientenliege herumdrehen. Der Hochspannungstransformator 10 wird insbesondere zu einer Übertragung von Leistung für den Betrieb der Röntgenröhre 20 und/oder des Röntgendetektors 41 verwendet. Grundsätzlich ist denkbar, dass mittels des Hochspannungstransformators 10 Daten übertragen werden.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

**Patentansprüche**

1. Schaltungsanordnung (30), aufweisend

   - eine Röntgenröhre (20), welche eine Kathode (21) und eine Anode (22) aufweist, und
   - einen Hochspannungstransformator (10), welcher
   - zumindest eine Primärwicklung (11.1),
   - zumindest eine erste Sekundärwicklung (12.1), welche einen ersten Schwingkreis (12.1.S) aufweist, wobei der erste Schwingkreis (12.1.S) eine erste Streuinduktivität ($L_{\sigma 1}$) und eine erste Wicklungskapazität ($C_{W1}$) aufweist, welche gemeinsam eine erste Resonanzfrequenz einstellen, und
   - zumindest eine zweite Sekundärwicklung (12.2) aufweist, welche einen zweiten Schwingkreis (12.2.S) aufweist, wobei der zweite Schwingkreis (12.2.S) eine zweite Streuinduktivität ($L_{\sigma 2}$) und eine zweite Wicklungskapazität ($C_{W2}$) aufweist, welche gemeinsam eine zweite Resonanzfrequenz einstellen,
   - wobei die erste Resonanzfrequenz und die zweite Resonanzfrequenz übereinstimmen,

- wobei sich die erste Wicklungskapazität ($C_{W1}$) und die zweite Wicklungskapazität ($C_{W2}$) betragsmäßig unterscheiden und
- wobei sich die erste Streuinduktivität ($L_{\sigma1}$) und die zweite Streuinduktivität ($L_{\sigma2}$) betragsmäßig unterscheiden,

**dadurch gekennzeichnet,**

- **dass** die zumindest eine erste Sekundärwicklung (12.1) mit der Kathode (21) elektrisch verbunden ist und
- **dass** die zumindest eine zweite Sekundärwicklung (12.2) mit der Anode (22) elektrisch verbunden ist.

2. Schaltungsanordnung (30) nach Anspruch 1, wobei sich im Betrieb in der zumindest einen ersten Sekundärwicklung (12.1) ein erster Laststrom (I1) einstellt, wobei sich in der zumindest einen zweiten Sekundärwicklung (12.1) ein zweiter Laststrom (I2) einstellt, wobei sich der erste Laststrom (I1) von dem zweiten Laststrom (I2) um einen Differenzstrom (10.IΔ) des Hochspannungstransformators (10) unterscheidet, welcher (10.IΔ) zu einem Differenzstrom (20.IΔ) der Röntgenröhre (20) proportional ist.

3. Schaltungsanordnung (30) nach einem der vorhergehenden Ansprüche, wobei die zumindest eine Primärwicklung (11.1) einen ersten Zylinder (11.Z) mit einer ersten Längsachse (11.L) bildet, wobei die zumindest eine erste Sekundärwicklung (12.1) und die zumindest eine zweite Sekundärwicklung (12.2) gemeinsam einen zweiten Zylinder (12.Z) mit einer zweiten Längsachse (12.L) bilden, wobei die erste Längsachse (11.L) parallel zur zweiten Längsachse (12.L) ist und wobei der erste Zylinder (11.Z) relativ zum zweiten Zylinder (12.Z) entlang der ersten Längsachse (11.L) versetzt angeordnet ist.

4. Schaltungsanordnung (30) nach einem der vorhergehenden Ansprüche, wobei sich ein Wicklungsdraht der zumindest einen ersten Sekundärwicklung (12.1) von einem Wicklungsdraht der zumindest einen zweiten Sekundärwicklung (12.2) gemäß zumindest einem Parameter der folgenden Liste unterscheidet:

- einer Dicke des Wicklungsdrahts,
- einer Dicke einer Lackisolationsschicht des Wicklungsdrahts,
- einer Dielektrizitätszahl der Lackisolationsschicht des Wicklungsdrahts.

5. Schaltungsanordnung (30) nach einem der vorhergehenden Ansprüche, wobei ein erster Durchmesser (12.1.D) der zumindest einen ersten Sekundärwicklung (12.1) kleiner ist als ein zweiter Durchmesser (12.2.D) der zumindest einen zweiten Sekundärwicklung (12.2), wodurch die erste Streuinduktivität ($L_{\sigma1}$) kleiner ist als die zweite Streuinduktivität ($L_{\sigma2}$).

6. Schaltungsanordnung (30) nach einem der vorhergehenden Ansprüche, wobei der Hochspannungstransformator (10) einen Primärspulenkörper und einen Sekundärspulenkörper aufweist, wobei der Sekundärspulenkörper eine erste Sekundärspulenhälfte (12.1.K) und eine zweite Sekundärspulenhälfte (12.2.K) aufweist, wobei die erste Sekundärspulenhälfte (12.1.K) die zumindest eine erste Sekundärwicklung (12.1) aufweist, wobei die zweite Sekundärspulenhälfte (12.2.K) die zumindest eine zweite Sekundärwicklung (12.2) aufweist und wobei die erste Sekundärspulenhälfte (12.1.K) und die zweite Sekundärspulenhälfte (12.2.K) asymmetrisch zueinander aufgebaut sind.

7. Schaltungsanordnung (30) nach Anspruch 6, wobei die zumindest eine erste Sekundärwicklung (12.1) in einer ersten Kammer (12.1.C) der ersten Sekundärspulenhälfte (12.1.K) gemeinsam mit zumindest einer ersten Papierisolationslage (12.1.P) angeordnet ist, wobei die zumindest eine zweite Sekundärwicklung (12.2) in einer zweiten Kammer (12.2.C) der zweiten Sekundärspulenhälfte (12.2.K) gemeinsam mit zumindest einer zweiten Papierisolationslage (12.2.P) angeordnet ist und wobei sich eine Anordnung der ersten Papierisolationslage (12.1.P) relativ zu der zumindest einen ersten Sekundärwicklung (12.1) von einer Anordnung der zweiten Papierisolationslage (12.2.P) relativ zu der zumindest einen zweiten Sekundärwicklung (12.2) unterscheidet.

8. Schaltungsanordnung (30) nach einem der Ansprüche 6 bis 7, wobei die zumindest eine erste Sekundärwicklung (12.1) in einer ersten Kammer (12.1.C) der ersten Sekundärspulenhälfte (12.1.K) angeordnet ist, wobei die zumindest eine zweite Sekundärwicklung (12.2) in einer zweiten Kammer (12.2.C) der zweiten Sekundärspulenhälfte (12.2.K) angeordnet ist und wobei eine erste Breite (12.1.B) der ersten Kammer (12.1.C) größer ist als eine zweite Breite (12.2.B) der zweiten Kammer (12.2.C), wodurch die erste Wicklungskapazität ($C_{W1}$) größer ist als die zweite Wicklungskapazität ($C_{W2}$).

9. Schaltungsanordnung (30) nach den Ansprüchen 5 und 8.

**10.** Schaltungsanordnung (30) nach einem der vorhergehenden Ansprüche, wobei die Röntgenröhre (20) ein Metallmittelteil (23) aufweist und wobei sich im Betrieb ein Differenzstrom (20.I$\Delta$) der Röntgenröhre (20) zwischen der Anode (22) und dem Metallmittelteil (23) einstellt.

**11.** Schaltungsanordnung (30) nach einem der vorhergehenden Ansprüche, wobei zumindest eine Schwingkreisdrossel zwischen dem Hochspannungstransformator (10) und der Röntgenröhre (20) verschaltet ist.

**12.** Schaltungsanordnung (30) nach einem der vorhergehenden Ansprüche, wobei zumindest ein Kondensator zwischen dem Hochspannungstransformator (10) und der Röntgenröhre (20) verschaltet ist.

**13.** Röntgeneinrichtung (40), aufweisend

   - einen Röntgendetektor (41) und
   - eine Schaltungsanordnung (30) nach einem der vorhergehenden Ansprüche.

**14.** Computertomograph, aufweisend

   - eine Röntgeneinrichtung (40) nach Anspruch 13.


**Claims**

**1.** Circuit arrangement (30), having

   - an X-ray tube (20), which has a cathode (21) and an anode (22), and
   - a high-voltage transformer (10), which
   - has at least one primary winding (11.1),
   - at least one first secondary winding (12.1), which has a first oscillating circuit (12.1.S), wherein the first oscillating circuit (12.1.S) has a first leakage inductance ($L_{\sigma 1}$) and a first winding capacitance ($C_{W1}$), which together adjust a first resonance frequency, and
   - at least one second secondary winding (12.2), which has a second oscillating circuit (12.2.S), wherein the second oscillating circuit (12.2.S) has a second leakage inductance ($L_{\sigma 2}$) and a second winding capacitance ($C_{W2}$), which together adjust a second resonance frequency,
   - wherein the first resonance frequency and the second resonance frequency match,
   - wherein the first winding capacitance ($C_{W1}$) and the second winding capacitance ($C_{W2}$) differ in value and
   - wherein the first leakage inductance ($L_{\sigma 1}$) and the second leakage inductance ($L_{\sigma 2}$) differ in value,

   **characterised in that**

   - the at least one first secondary winding (12.1) is electrically connected to the cathode (21) and
   - the at least one second secondary winding (12.2) is electrically connected to the anode (22).

**2.** Circuit arrangement (30) according to claim 1, wherein a first load current (II) is adjusted during operation in the at least one first secondary winding (12.1), wherein a second load current (12) is adjusted in the at least one second secondary winding (12.1), wherein the first load current (II) differs from the second load current (12) by a differential current (10.I$\Delta$) of the high-voltage transformer (10), which (10.I$\Delta$) is proportional to a differential current (20.I$\Delta$) of the X-ray tube (20).

**3.** Circuit arrangement (30) according to one of the preceding claims, wherein the at least one primary winding (11.1) forms a first cylinder (11.Z) having a first longitudinal axis (11.L), wherein the at least one first secondary winding (12.1) and the at least one second secondary winding (12.2) together form a second cylinder (12.Z) having a second longitudinal axis (12.L), wherein the first longitudinal axis (11.L) is parallel to the second longitudinal axis (12.L) and wherein the first cylinder (11.Z) is arranged offset relative to the second cylinder (12.Z) along the first longitudinal axis (11.L).

**4.** Circuit arrangement (30) according to one of the preceding claims, wherein a winding wire of the at least one first secondary winding (12.1) differs from a winding wire of the at least one second secondary winding (12.2) according to at least one parameter from the following list:

- a thickness of the winding wire,
- a thickness of an enamel insulation layer of the winding wire,
- a dielectric constant of the enamel insulation layer of the winding wire.

5. Circuit arrangement (30) according to one of the preceding claims, wherein a first diameter (12.1.D) of the at least one first secondary winding (12.1) is smaller than a second diameter (12.2.D) of the at least one second secondary winding (12.2), whereby the first leakage inductance ($L_{\sigma 1}$) is smaller than the second leakage inductance ($L_{\sigma 2}$).

6. Circuit arrangement (30) according to one of the preceding claims, wherein the high-voltage transformer (10) has a primary coil body and a secondary coil body, wherein the secondary coil body has a first secondary coil half (12.1.K) and a second secondary coil half (12.2.K), wherein the first secondary coil half (12.1.K) has the at least one first secondary winding (12.1), wherein the second secondary coil half (12.2.K) has the at least one second secondary winding (12.2) and wherein the first secondary coil half (12.1.K) and the second secondary coil half (12.2.K) are constructed asymmetrically to each other.

7. Circuit arrangement (30) according to claim 6, wherein the at least one first secondary winding (12.1) is arranged in a first chamber (12.1.C) of the first secondary coil half (12.1.K) together with at least one first paper insulation layer (12.1.P), wherein the at least one second secondary winding (12.2) is arranged in a second chamber (12.2.C) of the second secondary coil half (12.2.K) together with at least one second paper insulation layer (12.2.P) and wherein an arrangement of the first paper insulation layer (12.1.P) relative to the at least one first secondary winding (12.1) differs from an arrangement of the second paper insulation layer (12.2.P) relative to the at least one second secondary winding (12.2).

8. Circuit arrangement (30) according to one of claims 6 to 7, wherein the at least one first secondary winding (12.1) is arranged in a first chamber (12.1.C) of the first secondary coil half (12.1.K), wherein the at least one second secondary winding (12.2) is arranged in a second chamber (12.2.C) of the second secondary coil half (12.2.K) and wherein a first width (12.1.B) of the first chamber (12.1.C) is greater than a second width (12.2.B) of the second chamber (12.2.C), whereby the first winding capacitance ($C_{W1}$) is greater than the second winding capacitance ($C_{W2}$).

9. Circuit arrangement (30) according to claims 5 and 8.

10. Circuit arrangement (30) according to one of the preceding claims, wherein the X-ray tube (20) has a metal central part (23) and wherein a differential current (20.I$\Delta$) of the X-ray tube (20) is adjusted between the anode (22) and the metal central part (23) during operation.

11. Circuit arrangement (30) according to one of the preceding claims, wherein at least one oscillating circuit throttle is interconnected between the high-voltage transformer (10) and the X-ray tube (20).

12. Circuit arrangement (30) according to one of the preceding claims, wherein at least one capacitor is interconnected between the high-voltage transformer (10) and the X-ray tube (20) .

13. X-ray unit (40), having

- an X-ray detector (41) and
- a circuit arrangement (30) according to one of the preceding claims.

14. Computed tomography unit, having

- an X-ray unit (40) according to claim 13.


**Revendications**

1. Montage (30), comportant

- un tube (20) à rayons X, qui a une cathode (21) et une anode (22), et
- un transformateur (10) de haute tension, qui a
- au moins un enroulement (11.1) primaire,

- au moins un premier enroulement (12.1) secondaire, qui a un premier circuit (12.1.S) oscillant, le premier circuit (12.1.S) oscillant ayant une première inductance ($L_{\sigma 1}$) de fuite et une première capacité ($C_{W1}$) d'enroulement, qui établissent conjointement une première fréquence de résonnance, et

- au moins un deuxième enroulement (12.2) secondaire, qui a un deuxième circuit (12.2.S) oscillant, le deuxième circuit (12.2.S) oscillant ayant une deuxième inductance ($L_{\sigma 2}$) de fuite et ne deuxième capacité ($C_{W2}$) d'enroulement, qui établissent conjointement une deuxième fréquence de résonnance,

- dans lequel la première fréquence de résonnance et la deuxième fréquence de résonnance coïncident,

- dans lequel la première capacité ($C_{W1}$) d'enroulement et la deuxième capacité ($C_{W2}$) d'enroulement sont différentes en valeur absolue et

- dans lequel la première inductance ($L_{\sigma 1}$) de fuite et la deuxième inductance ($L_{\sigma 2}$) de fuite sont différentes en valeur absolue,

**caractérisé**

- **en ce que** le au moins un premier enroulement (12.1) secondaire est connecté électriquement à la cathode (21) et

- **en ce que** le au moins un deuxième enroulement (12.2) secondaire est connecté électriquement à l'anode (22).

2. Montage (30) suivant la revendication 1, dans lequel, en fonctionnement, le au moins un premier enroulement (121 ) secondaire établit un premier courant (I1) de charge, un deuxième courant (I2) de charge s'établissant dans le au moins un deuxième enroulement (12.1) secondaire, le premier courant (I1) de charge se distinguant du deuxième courant (I2) de charge par un courant (10.i$\Delta$) de différence du transformateur (10) de haute tension, qui (10.i$\Delta$) est proportionnel à un courant (20.i$\Delta$) de différence du tube (20) à rayons X.

3. Montage (30) suivant l'une des revendications précédentes , dans lequel le au moins un enroulement (11.1) primaire forme un premier cylindre (11.Z) ayant un premier axe (11.L) longitudinal, le au moins un premier enroulement (12.1) secondaire et le au moins un deuxième enroulement (12.2) secondaire formant conjointement un deuxième cylindre (12.Z) ayant un deuxième axe (12.L) longitudinal, le premier axe (11.L) longitudinal étant parallèle au deuxième axe (12.L) longitudinal et le premier cylindre (11.Z) étant disposé de manière décalée le long du premier axe (11.L) longitudinal par rapport au deuxième cylindre (12.Z).

4. Montage (30) suivant l'une des revendications précédentes, dans lequel un fil d'enroulement du au moins un premier enroulement (12.1) secondaire se distingue d'un fil d'enroulement du au moins un deuxième enroulement (12.2) secondaire par au moins un paramètre de la liste suivante :

- une épaisseur du fil d'enroulement,
- une épaisseur d'une couche isolante de vernis du fil d'enroulement,
- un facteur de permittivité de la couche isolante en vernis du fil d'enroulement.

5. Montage (30) suivant l'une de revendications précédentes, dans lequel un premier diamètre (12.1.D) du au moins un premier enroulement (12.1) secondaire est plus petit qu'un deuxième diamètre (12.2.D) du au moins un deuxième enroulement (12.2) secondaire, grâce à quoi la première inductance ($L_{\sigma 1}$) de fuite est plus petite que la deuxième inductance ($L_{\sigma 2}$) de fuite.

6. Montage (30) suivant l'une des revendications précédentes, dans lequel le transformateur (10) de haute tension a un mandrin primaire et un mandrin secondaire, le mandrin secondaire ayant une première moitié (12.1.K) de mandrin secondaire et une deuxième moitié (12.2.K) de mandrin secondaire, la première moitié (12.1.K) de mandrin secondaire ayant le au moins un premier enroulement (12.1) secondaire, la deuxième moitié (12.2.K) de mandrin secondaire ayant le au moins un deuxième enroulement (12.2) secondaire et la première moitié (12.1.K) de mandrin secondaire et la deuxième moitié (12.2.K) de mandrin secondaire étant constituées de manière dissymétrique l'une de l'autre.

7. Montage (30) suivant la revendication 6, dans lequel le au moins un premier enroulement (12.1) secondaire est disposé dans une première chambre (12.1.C) de la première moitié (12.1.K) de mandrin secondaire, conjointement avec au moins une première couche (12.1.P) isolante en papier, le au moins un deuxième enroulement (12.2) secondaire étant disposé dans une deuxième chambre (12.2.C) de la deuxième moitié (12.2.K) de mandrin secondaire, conjointement avec au moins une deuxième couche (12.2.P) isolante en papier et dans lequel un agencement de la première couche (12.1.P) isolante en papier, par rapport au au moins un premier enroulement (12.1) secondaire, étant différent d'un agencement de la deuxième couche (12.2.P) isolante en papier, par rapport au au moins un deuxième enroulement (12.2) secondaire.

8. Montage (30) suivant l'une des revendications 6 à 7, dans lequel le au moins un premier enroulement (12.1) secondaire est disposé dans une première chambre (12.1.C) de la première moitié (12.1.K) de mandrin secondaire, au moins un deuxième enroulement (12.2) secondaire étant disposé dans une deuxième chambre (12.2.C) de la deuxième moitié (12.2.K) du mandrin secondaire et une première largeur (12.1.B) de la première chambre (12.1.C) étant plus grande qu'une deuxième largeur (12.2.B) de la deuxième chambre (12.2.C), grâce à quoi la première capacité ($C_{W1}$) d'enroulement est plus grande que la deuxième capacité ($C_{W2}$) d'enroulement.

9. Montage (30) suivant les revendications 5 et 8.

10. Montage (30) suivant l'une des revendications précédentes, dans lequel le tube (20) à rayons X a une partie (23) médiane métallique, et dans lequel, en fonctionnement, un courant (20.IΔ) de différence du tube (20) à rayons X s'établit entre l'anode (22) et la partie (23) médiane métallique.

11. Montage (30) suivant l'une des revendications précédentes, dans lequel au moins une bobine de circuit oscillant est montée entre le transformateur (10) de haute tension et le tube (20) à rayons X.

12. Montage (30) suivant l'une des revendications précédentes, dans lequel au moins un condensateur est monté entre le transformateur (10) de haute tension et le tube (20) à rayons X.

13. Dispositif (40) radiologique, comportant

- un détecteur (41) de rayons X et
- un montage (30) suivant l'une des revendications précédentes.

14. Tomographe assisté par ordinateur, comportant

- un dispositif (40) radiologique suivant la revendication 13.

FIG 1

FIG 2

FIG 3

FIG 4

EP 3 562 275 B1

## FIG 5

## FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4481654 A